(19) Europäisches Patentamt
European Patent Office
Office européen des brevets

(11) Veröffentlichungsnummer: **0 000 505**
**B1**

(12) # EUROPÄISCHE PATENTSCHRIFT

(21) Anmeldenummer: **78100373.6**

(22) Anmeldetag: **12.07.78**

(51) Int. Cl.³: **C 07 D 213/69,**
C 09 B 55/00, C 08 K 5/34

(54) **Metallkomplexe, Verfahren zu ihrer Herstellung und ihre Verwendung als Pigmente**

(30) Priorität: **29.07.77 US 819756**

(43) Veröffentlichungstag der Anmeldung:
**07.02.79 Patentblatt 79/03**

(45) Bekanntmachung des Hinweises auf die Patenterteilung:
**03.09.80 Patentblatt 80/18**

(84) Benannte Vertragsstaaten: **BE CH DE FR GB**

(56) Entgegenhaltungen:
**DE - A - 2 008 938**
**FR - A - 2 163 519**
**HOUBEN-WEYL, BAND I/2,**
**Seite 325, 326**

(73) Patentinhaber: **Cassella Aktiengesellschaft**
**Hanauer Landstrasse 526 D - 6000**
**Frankfurt am Main 61 (DE)**

(72) Erfinder: **Tappe, Horst, Dr.**
**Ringstrasse 9 D - 6057 Dietzenbach (DE)**

(74) Vertreter: **Urbach, Hans-Georg, Dr.**
**Hanauer Landstrasse 526 D - 6000**
**Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 505 B1

Metallkomplexe, Verfahren zu ihrer Herstellung und ihre Verwendung als Pigmente

Die Erfindung betrifft wertvolle Metallkomplexe der Formel I

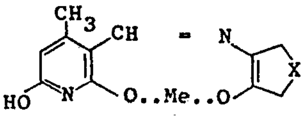

(I)

worin X für

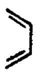 , 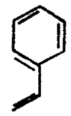 , 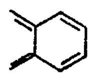 oder 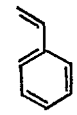

steht und der durch X mit dem übrigen Teil des Moleküls gebildete Benzol- oder Naphthalinkern auch durch ein oder zwei Substituenten aus der Reihe Methyl, Methoxy, Chlor, Brom, Trifluormethyl, Nitro, Cyan, $CONR^1R^2$ oder $COOR^3$, wobei $R^1$ und $R^2$ für Wasserstoff oder Alkyl mit 1 bis 4 C-Atomen und $R^3$ für Alkyl mit 1 bis 4 C-Atomen stehen, substituiert sein kann, und Me ein Nickel-, Kupfer-, Zink-oder Kobaltatom bedeutet. Die Erfindung betrifft ferner die Verwendung der Metallkomplexe als Pigmente und zur Pigmentierung von Lacken, hochmolekularen plastischen Massen und Druckpasten.

Metallkomplexe der Formel II

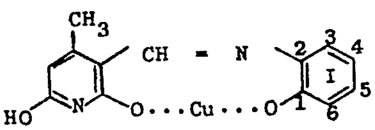

(II)

wobei der Benzolkern I auch noch wie vorstehend angegeben, insbesondere einfach in para-Stellung zum Sauerstoff, d.h.in 4-Stellung, substituiert sein kann, sind von besonderem Interesse.

Die neuen Metallkomplexe können dadurch hersetellt werden, daß man 3 - Formyl - 4 - methyl - 2,6 - dihydroxy - pyridin bzw. ein Alkali- oder Erdalkalisalz davon mit einer ortho-Aminohydroxyverbindung der Formel III

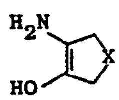

(III)

worin X die oben genannte Bedeutung besitzt und ein Benzol-oder Naphthalinkern, wie vorstehend angegeben, substituiert sein kann, nach an sich bekannten Methoden umsetzt und das entstehende Kondensationsprodukt nach einer an sich bekannten Methode metallisiert.

Als Ausgangsprodukte sind z.B. folgende ortho - Aminohydroxy - verbindungen geeignet: ortho-Aminophenol, 2 - Amino - 4 - (oder 3- oder 5-oder 6-)nitrophenol, 2 - Amino - 4 - (oder 3-oder 5-oder 6-)chloro - phenol, 2 - Amino - 4 - (oder 3- oder 5-oder 6-)bromo - phenol, 2 - Amino - 4 - (oder 3- oder 5- oder 6-)cyano - phenol, 2 - Amino - 4 - (oder 3- oder 5- oder 6-) methoxycarbonylphenol, 2 - Amino - 4 - (oder 3- oder 5- oder 6-)äthoxycarbonylphenol, 2-Amino - 4 - (oder 3-)butoxycarbonyl-phenol, 2 - Amino - 4 - (oder 5-) trifluormethyl - phenol, 2 - Amino - 4 - (oder 5-) - carbonamido - phenol, 2 - Amino - 4- (oder 5-)dimethylcarbonamido - phenol, 2 - Amino - 4 - (oder 5-) äthylcarbonamido - phenol, 2 - Amino - 1 - naphthol, 1 - Amino - 2 - naphthol, 2 - Amino - 3 - naphthol, 2 - Amino - 4 - (oder 5 - oder 7 -)chloro - 1 - naphthol, 1 - Amino - 4 - (oder 5-, 6- oder 7-)chloro - 2 - naphthol, 1 - Amino - 3 - (oder 4- oder 8-)nitro - 2 - naphthol.

Die Verbindungen der Formel III sind zumeist bekannt bzw. können leicht nach den für die Herstellung von ortho - Amino - hydroxyverbindungen bekannten Methoden synthetisiert werden. Das 3 - Formyl - 4 - methyl - 2,6 - dihydroxy - pyridin und seine Alkali - oder Erdalkalisalze sind neu. Es kann leicht, wie in den nachfolgenden Beispielen beschrieben, hergestellt werden. Von dem 3 - Formyl - 4 - methyl - 2,6 - dihydroxy - pyridin sind auch andere tautomere Formen, z.B. das 2 - Hydroxy - 3 - formyl - 4 - methylpyridon - (6) denkbar. Von seinen Salzen sind besonders das Natrium- und Kaliumsalz geeignet. Auch von den Metallkomplexen der allgemeinen Formel I sind noch andere tautomere Formen denkbar. Selbstverständlich wird von der vorliegenden Erfindung jede der möglichen tautomeren Formen umfaßt. Die Umsetzung des 3-Formyl-4-methyl-2,6-dihydroxy-pyridins oder des entsprechenden Alkali- oder Erdalkalisalzes mit der ortho-Amino-hydroxy-Verbindung wird in geeigneten organischen Lösungsmitteln, beispielsweise Alkoholen mit 1 bis 5 C-Atomen, Glykoläthern, wie z.B. Äthylenglykoldimethyläther, Eisessig, aprotischen Lösungsmitteln, wie z.B. Toluol, Tetrachlorkohlenstoff, 1,4-Dioxan, Tetrahydrofuran und dergleichen, insbesondere dipolar aprotischen Lösemitteln, wie z.B. Dimethylformamid, Dimethylsulfoxid, N-Methylpyrolidon, Tetramethylensulfon und dergleichen oder Wasser oder Gemischen davon, zweckmäßigerweise unter einer Atmosphäre eines Inertgases, wie Stickstoff, bei erhöhten Temperaturen durchgeführt. Als Reaktionstemperaturen können Temperaturen von 60°C bis zur Rückflußtemperatur des Lösungsmittels in Betracht kommen. Die bevorzugten Reaktionstemperaturen betragen von 80 bis 120°C. Die Umsetzung ist normalerweise innerhalb von 2 bis 8 Stunden beendet. Das bei dieser Umsetzung entstandene Azomethin kann ohne Isolierung metallisiert werden, aber auch eine Isolierung des Azomethins vor der Metallisierung ist möglich. Es genügt jedoch im allgemeinen, das Azomethin ohne Isolierung in einem geeigneten Lösungsmittel zu metallisieren. Zu diesem Zweck wird das metallfreie Azomethin in dem zur Azomethinkondensation benutzten Lösungsmittel oder Lösungsmittelgemisch mit einem Metallsalz, vorzugsweise einem Acetat, z.B. Kupfer - II - acetat, unter den gleichen Bedingungen wie bei der Azomethinherstellung umgesetzt. Dabei kann es zweckmäßig sein, den pH-Wert der Lösung zwischen 5 und 6 zu halten, was z.B. durch sukzessive Zugabe von Natriumacetatlösung erfolgen kann.

Die metallfreien Azomethine sind gelborangefarbene bis braune Produkte, während die metallhaltigen neuen Metallkomplexe normalerweise gelbstichig-grün bis olivgrün und in den üblichen Lösungsmitteln nur schwer bis sehr schwer löslich sind. Die neuen Metallkomplexe eignen sich daher als farbstarke lösungs- und temperaturbeständige, besonders aber hervorragend licht- und wetterechte Pigmente zur Herstellung von Druckfarben, zum Färben von Kunststoffen in der Masse und als Spinnfarbstoffe und insbesondere zur Herstellung von Lacken und Anstrichstoffen. Aufgrund der guten Echtheiten sind die Pigmente besonders auch in Form ihrer Metallic-Lacke (Aluminium-Lacke) für hochwertige Außen-(Auto-)lacke interessant.

Die metallfreien und metallhaltigen Azomethine weisen im Pyridinkern eine freie Stellung auf, an die noch Diazokomponenten angekuppelt werden können. So ist es möglich, wertvolle metallfreie und metallhaltige Azomethine herzustellen, die noch eine Azogruppe enthalten.

Wenn nichts anderes angegeben ist, sind in den nachfolgenden Beispielen Teile in Gewichtsteilen und Temperaturen in Grad Celcius angegeben.

Beispiel 1

21,1 g 2 - Hydroxy - 3 - formyl - 4 - methylpyridon - (6) als Kaliumsalz werden zusammen mit 14,9 g 4 - Chlor - 2 - aminophenol in 300 ml Eisessig suspendiert und unter Stickstoffatmosphäre 3 Stunden zum Sieden erhitzt (ca. 118°C). Dann werden 10 g Kupfer - II - acetat, gelöst in 300 ml Dimethylformamid, zugegeben, und es wird eine Stunde bei 140°C gehalten. Nach Abkühlen wird abgesaugt, mit Methanol und Wasser gewaschen und getrocknet. Man erhält 33,5 g eines gelbstichig-grünen Metallkomplexes der Formel

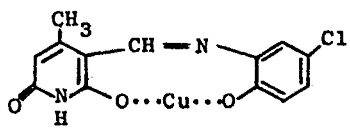

der bei seiner Verwendung als Pigmente, z.B. in Metallic-(Aluminium)-Autolacken, hervorragende Licht- und Wetterechtheiten zeigt.

Beispiel 2

1200 Teile Wasser werden unter Einleiten von Stickstoff zum Sieden erhitzt und unter Stickstoffeinleitung auf 20°C abgekühlt. Dann gibt man 57,3 Teile 2 - Hydroxy - 3 - formyl - 4 - methylpyridon-(6) und 3,4,5 Teile o-Aminophenol zu, erhitzt 4 Stunden am Rückfluß, saugt heiß ab und wäscht mit 1000 Teilen 80°C heißem Wasser. Die erhaltene gelbe Paste des Azomethins gibt man in 6000 Teile Wasser, fügt 60 Teile Kupfer - II - acetat zu und erhitzt 3 1/2 Stunden am Rückfluß, wobei man durch sukzessive Zugabe von 1100 Teilen 10%iger Natrium-acetat-Lösung den pH-Wert zwischen 5 und 6 hält. Am Ende der Reaktion beträgt der pH-Wert 5,4. Das Produkt wird heiß abgesaugt und mit 1200 Teilen 80°C heißem Wasser gewaschen und bei 40°C im Vakuum getrocknet. Man erhält 44 Teile des Azomethin-Kupfer-Komplexes der Formel

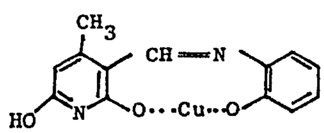

Das erhaltene olivgrüne Pigment kann in üblicher Weise zu einer Druckfarbe verarbeitet werden, die farbstarke Drucke von hervorragender Lichtechtheit liefert.

Ein unter Verwendung des erhaltenen Pigments in üblicher Weise hergestellter Autoeinbrennlack zeigte nach dem Einbrennen sehr gute Lichtechtheit, Wetterrechtheit und Überspritzechtheit.

Ähnlich wie im vorstehenden Beispiel 2 wurden die in den nachfolgenden Tabellen angegebenen Metallkomplexe hergestellt:

TABELLE 1

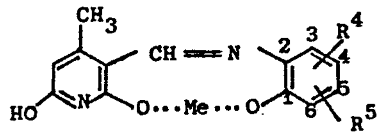

| $R^4$ | $R^5$ | Me | $R^4$ | $R^5$ | Me |
|---|---|---|---|---|---|
| 4-Cl | H | Cu | 3-$CF_3$ | H | Co |
| 4-Br | H | Cu | 3-$CF_3$ | H | Zn |
| 4-$NO_2$ | H | Cu | 5-$CF_3$ | H | Cu |
| 4-$CONH_2$ | H | Cu | 6-$CF_3$ | H | Cu |
| 4-$CONHCH_3$ | H | Cu | 5-$COOCH_3$ | H | Cu |
| 4-$CONHC_2H_5$ | H | Cu | 5-$COOCH_3$ | H | Zn |
| 4-$CONHC_4H_9$(n) | H | Cu | 5-$COOC_2H_5$ | H | Ni |
| 4-$COOCH_3$ | H | Cu | 3-Cl | 5-$COOCH_3$ | Cu |
| 4-$COOC_2H_5$ | H | Cu | 6-Br | 4-$COOC_2H_5$ | Cu |
| 4-$COOC_3H_7$(n) | H | Cu | 4-$CH_3$ | H | Cu |
| 4-$COOC_4H_9$(n) | H | Cu | 4-$OCH_3$ | H | Cu |
| 4-$CF_3$ | H | Cu | 4-CN | H | Cu |
| 3-Cl | 5-Cl | Cu | H | H | Co |
| 3-Br | 5-Br | Cu | H | H | Ni |
| 4-Cl | H | Co | H | H | Zn |
| 4-Br | H | Co | | | |
| 4-Cl | H | Ni | | | |
| 4-Br | H | Ni | | | |

TABELLE 2

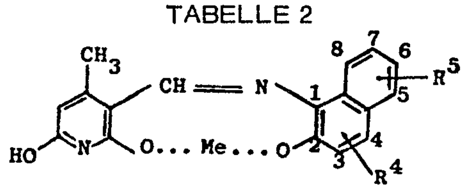

| $R^4$ | $R^5$ | Me | $R^4$ | $R^5$ | Me |
|---|---|---|---|---|---|
| 3-Br | H | Cu | 4-Br | H | Ni |
| 3-Cl | H | Cu | 4-$NO_2$ | 8-Br | Zn |
| 4-Br | H | Cu | 4-$CH_3$ | H | Cu |
| 4-Cl | H | Cu | 4-$OCH_3$ | H | Cu |
| 4-$NO_2$ | H | Cu | H | H | Cu |
| 4-Cl | H | Co | H | H | Co |
| | | | H | H | Ni |

TABELLE 3

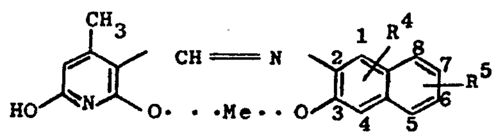

| $R^4$ | $R^5$ | Me | $R^4$ | $R^5$ | Me |
|---|---|---|---|---|---|
| H | H | Cu | 8-Br | H | Cu |
| H | H | Co | 8-$NO_2$ | H | Ni |
| H | H | Zn | 8-Cl | 4-Cl | Cu |

TABELLE 4

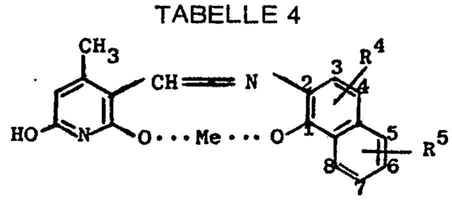

| $R^4$ | $R^5$ | Me | $R^4$ | $R^5$ | Me |
|---|---|---|---|---|---|
| H | H | Cu | 4-Br | H | Cu |
| H | H | Co | 4-Br | 8-Br | Co |
| H | H | Zn | 3-$NO_2$ | H | Cu |

Das in den vorstehenden Beispielen als Ausgangsprodukt benutzte 6 - Hydroxy - 3 - formyl - 4 - methylpyridon - (6) bzw. sein Natriumsalz kann, ausgehend vom 4 - Methyl - 2,6 - dihydroxypyridin, folgendermaßen hergestellt werden:

125 Teile 4 - Methyl - 2,6 - dihydroxypyridin in 400 Teilen Äthanol werden mit 80 Teilen Natriumacetat, gelöst in 100 Teilen Wasser, und 148 Teilen Chloral in 400 Teilen Wasser zusammengegeben und das Gemisch 8 Stunden bei 20°C gerührt, anschließend läßt man weitere 48 Stunden bei Zimmertemperatur stehen, saugt dann das ausgefallene Produkt ab, wäscht den Rückstand mit Wasser und trocknet bei 30°C im Vakuum. Man erhält so 218 Teile (=80% d.Th.) 3 - ($\beta,\beta,\beta$ - Trichlor - $\alpha$ - hydroxy) - äthyl - 4 - methyl - 2,6 - dihydroxypyridin, das nach Umkristallisation aus Essigsäureäthylester einen Schmelzpunkt von 195°C besitzt.

| | | |
|---|---|---|
| Analyse: berechnet: | N 5,4% | Cl 39,9% |
| gefunden: | N 5,1% | Cl 39,1% |

272,5 Teile 3-($\beta,\beta,\beta$ - Trichlor - $\alpha$ - hydroxy)äthyl - 4 - methyl - 2,6 - dihydroxypyridin werden in 3000 Teile Äthanol eingetragen und mit einer Lösung von 500 Teilen Kaliumhydroxid in 400 Teilen Wasser und 1700 Teilen Äthanol versetzt. Das Reaktionsgemisch wird 30 Minuten am Rückfluß unter Rühren am Sieden gehalten, dann auf 20°C abgekühlt. Das hierbei ausgeschiedene Produkt wird abgesaugt, mit 200 Teilen Äthanol gewaschen und getrocknet. Das so erhaltene Rohprodukt kann durch Waschen bis zur neutralen Reaktion mit verdünnter Essigsäure von anhaftender überschüssiger Kalilauge befreit werden. Man erhält so 94 Teile des Kalium - 3 - formyl - 4 - methyl - 2,6 - dihydroxypyridins (= 49% d.Th.), das nach Umkristallisation aus Wasser einen Schmelzpunkt von über 350°C besitzt.

| | | | |
|---|---|---|---|
| Analyse: berechnet: | C 44,0% | H 3,2% | N 7,3% |
| gefunden: | C 44,2% | H 3,4% | N 7,3% |

Verwendet man anstelle von Kaliumhydroxid Natriumhydroxid, so erhält man das entsprechende Natriumsalz.

Die Überführung des Kalium- oder Natriumsalzes in die Metallfreie Verbindung geschieht in der Weise, daß 19,1 Teile des erhaltenen Produkts in 200 Teilen eines Gemischs aus 160 Teilen Essigsäure und 40 Teilen Wasser suspendiert werden und das Reaktionsgemisch im Vakuum auf 50 Teile eingeengt wird. Nach Stehenlassen der eingeengten Lösung kristallisiert die metallfreie Verbindung aus. Durch Umlösen und Umkristallisieren aus einem Gemisch aus 160 Teilen Äthanol und 40 Teilen Wasser erhält man das reine 3 - Formyl - 4 - methyl - 2,6 - dihydroxypyridin vom Schmelzpunkt 180 bis 182°C.

| | | | |
|---|---|---|---|
| Analyse: berechnet: | C 54,9% | H 4,6% | N 9,1% |
| gefunden: | C 55,1% | H 4,7% | N 9,0% |

## Beispiel 3

4,5 g des nach Beispiel 1 erhaltenen Metallkomplexes werden mit 25,5 g eines Anreibelacks, bestehend aus einer 20%igen Lösung von Alkyldharzlack in Xylol auf der Vibrationskugelmühle 45 Minuten geschüttelt, mit 60 g Klarlack, bestehend aus 52,5 g einer 70%igen Alkydharzlösung in Xylol, 35 g einer 55%igen Melaminharzlösung in Butanol, 2,5 g Butylglykol, 5 g Butanol und 5 g Lackbenzin versetzt. Man erhält so einen klaren gelbstichig-grünen Volltonlack, der auf ein Aluminiumblech gespritzt und 30 Minuten bei 140°C eingebrannt eine gelbstichig-grüne Färbung von einwandfreier Überlackierechtheit und ausgezeichneter Licht- und Wetterechtheit ergibt.

## Beispiel 4

Eine Mischung von 62,5 ml Glasperlen, 3 mm Durchmesser, 4,5 g des nach Beispiel 2 erhaltenen Metallkomplexes und 25,2 g Acryl Anreibelack 25%ig wird in einem verschlossenen Plastikbecher von 125 ml Inhalt 30 Minuten auf dem Paintshaker geschüttelt. Anschließend werden 60,3 g eines 48%igen Acryl-Melaminharz-Klarlacks zugemischt und sodann die Glaskugeln durch Sieben entfernt.

Der so hergestellte in bezug auf das organische Pigment 5%ige Volltonlack wird mit einem 3% Aluminium-Pigment enthaltenden Acryl-Melaminharzlack im Gewichtsverhältnis 3 : 5 vermischt.

Anschließend wird mit Xylol, Butanol and Solvesso 100, ein aus mehreren Aromaten bestehendes Lösemittelgemisch, (1 : 2,3 : 3,3) auf Spritzviskosität 4″ (Viskospatel) eingestellt und dieser Metalleffektlack auf ein mit einem Washprimer und einem Einbrennweißlack wie üblich grundiertes Blech mit der Spritzpistole (Düse 1,5 mm Durchmesser, Spritzdruck 4 bar) in 6 waagerechten Gängen aufgebracht.

Nach dem Ablüften wird 30 Minuten bei 140°C im Trockenschrank eingebrannt. Die Echtheiten der Lackierung, insbesondere die Licht- und Wetterechtheiten, sind hervorragend.

Beispiel 5

Eine Mischung von 62,5 ml Glasperlen, 3 mm Durchmesser, 3,8 g Metallkomplexe nach Beispiel 1 und 27,4 g Alkyd-Anreibelack 25 %ig wird in einem verschlossenen Plastikbecher von 125 ml Inhalt 60 Minuten auf dem Paintshaker geschüttelt. Anschließend werden 44,8 g eines 56%igen Alkyd-Melamin-harz-Klarlacks zugemischt und sodann die Glaskugeln durch Sieben entfernt. Der so hergestellte 5 Gewichtsprozente Pigment enthaltende Vollton-Farblack wird mittels eines Handcoaters in einer Naßfilmstärke von 75 $\mu$m auf ein weißes Kärtchen mit schwarzem Querstreifen aufgezogen. Nach dem Ablüften wird 30 Minuten bei 140°C im Trockenschrank eingebrannt. Es resultiert ein farbstarker gelbstichig-grüner Überzug mit hervorragenden Echtheiten.

Beispiel 6

2 Gewichtsteile eines 5 Gewichtsprozente Pigment enthaltenden, durch 60 Minuten langes Anreiben auf dem Paintshaker gemäß Beispiel 5 hergestellten Alkyd-Melaminharz-Einbrennlacks werden mit 2,5 Gewichtsteilen eines Einbrennweißlacks vermischt, der ebenfalls auf Alkyd-Melaminharz-Basis 20 Gewichtsprozente $TiO_2$ enthält.

Diese Weißaufhellung wird mit Hilfe eines Handcoaters in einer Naßfilmstärke von 75 $\mu$m auf ein weißes Kärtchen aufgezogen und der Aufstrich nach dem Ablüften 30 Minuten bei 140°C im Trockenschrank eingebrannt.

Beispiel 7

Von dem in Tabelle 1 in der Seitenzeile 14 aufgeführten Metallkomplex mit $R^4 = 4{-}COOCH_3$, $R^5 = H$ und Me = Cu wurde gemäß der Vorschrift des Beispiels 4 ein Metalleffektlack auf ein Blech aufgetragen und gemäß den Beispielen 5 und 6 ein 5%iger Volltonlack und ein mit Titandioxid 1 : 5 aufgehellter Lack auf weiße Kärtchen aufgetragen.

Zum Vergleich wurden entsprechende Lackierungen mit dem Metallkomplex

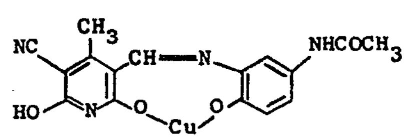

gemäß der FR—PS 21 63 519 angefertigt. Die mit dem erfindungsgemäßen Metallkomplex angefertigten Lackierungen sind den Vergleichslackierungen hinsichtlich Farbton und Reinheit deutlich überlegen. Bei einer 1000 stündigen Bewetterung der lackierten Bleche im Wechsellauf in einem Xenotest × 1200-Gerät zeigte der mit dem erfindungsgemäßen Metallkomplex hergestellte Metalleffektlack eine um 2 Noten bessere Echtheit gegenüber dem mit dem bekannten Metallkomplex hergestellten Metalleffektlack.

**Patentansprüche**

1. Metallkomplexe der Formel I

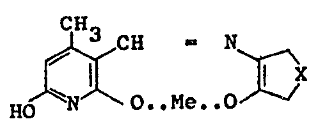

(I)

worin X für

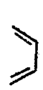 , 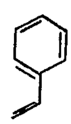 , 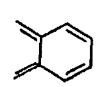 oder 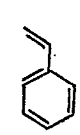

steht und der durch X mit dem übrigen Teil des Moleküls gebildete Benzol- oder Naphthalinkern auch durch ein oder zwei Substituenten aus der Reihe Methyl, Methoxy, Chlor, Brom, Trifluormethyl, Nitro, Cyan, $CONR^1R^2$ oder $COOR^3$, wobei $R^1$ und $R^2$ für Wasserstoff oder Alkyl mit 1 bis 4 C-Atomen und $R^3$ für Alkyl mit 1 bis 4 C-Atomen stehen, substituiert sein kann, und Me ein Nickel-, Kupfer-, Zink- oder Kobaltatom bedeutet.

2. Metallkomplexe der Formel II

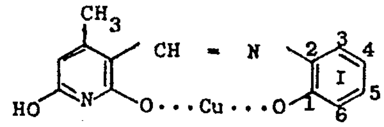

(II)

wobei der Benzolkern I wie in Anspruch 1 angegeben, subtituiert sein kann.

3. Metallkomplexe nach Anspruch 2, dadurch gekennzeichnet, daß der Benzolkern I unsubstituiert ist.

4. Metallkomplexe nach den Ansprüchen 1 und 2, dadurch gekennzeichnet, daß der Benzolkern I in 4-Stellung substituiert ist.

5. Metallkomplexe nach den Ansprüchen 1 bis 2 und 4, dadurch gekennzeichnet, daß der Benzolkern durch Chlor, Brom, Nitro oder Methoxycarbonyl substituiert ist.

6. Verwendung der Metallkomplexe nach den Ansprüchen 1 bis 5 als Pigmente.

7. Verwendung der Metallkomplexe nach den Ansprüchen 1 bis 5 zum Pigmentieren von Lacken.

8. Verfahren zur Herstellung der Metallkomplexe nach den Ansprüchen 1 bis 5, dadurch gekennzeichnet, daß 3-Formyl-4-methyl-2,6-dihydroxy-pyridin oder eines seiner Alkali-oder Erdalkalisalze mit einer ortho-Amino-hydroxy-verbindung der Formel

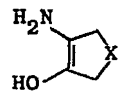

worin X die in Anspruch 1 genannte Bedeutung besitzt und der durch X mit dem übrigen Teil des Moleküls gebildete Benzol oder Naphthalinkern auch, wie in den Ansprüchen 1 bis 5 angegeben, substituiert sein kann, umgesetzt und das entstandene Kondensationsprodukt metallisiert wird.

9. Verfahren nach Anspruch 8, dadurch gekennzeichnet, daß die Umsetzung und/oder Metallisierung in einem organischen Lösungsmittel oder Wasser bei Temperaturen von 60°C bis zur Rückflußtemperatur des Lösungsmittels durchgeführt wird.

10. Verfahren nach den Ansprüchen 8 oder 9, dadurch gekennzeichnet, daß die Umsetzung und/oder Metallisierung unter Inertgasatmosphäre durchgeführt wird.

11. Verfahren nach den Ansprüchen 8 bis 10, dadurch gekennzeichnet, daß bei der Metallisierung ein pH-Wert zwischen 5 und 6 eingehalten wird.

**Revendications**

1. Complexes métalliques qui répondent à la formule I

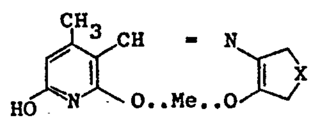

(I)

dans laquelle X représente

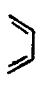 , 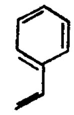 , 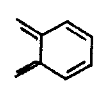 ou 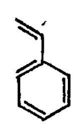

et le noyau bezénique ou naphtalénique formé par X avec la partie restante de la molécule peut également porter un ou deux substituants pris dans l'ensemble constitué par les radicaux méthyle et méthoxy, les atomes de chlore et de brome, le radical trifluorométhyle, les groupes nitro et cyano, et les radicaux —$CONR^1R^2$ et —$COOR^3$ dans lesquels $R^1$ et $R^2$ représentent chacun un atome d'hydrogène ou un alkyle contenant de 1 à 4 atomes de carbone et $R^3$ représente un alkyle contenant de 1 à 4 atomes de carbone, et Me représente un atome de nickel, de cuivre, de zinc ou de cobalt.

2. Complexes métalliques répondant à la formule II

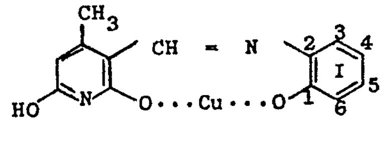

(II)

dans laquelle le noyau benzénique I peut être substitué comme indiqué à la revendication 1.

3. Complexes métalliques selon les revendications 1 et 2, caractérisés en ce que le noyau porte pas de substituants.

4. Complexes métalliques selon les revendications 1 et 2, caractérisés en ce que le noyau benzénique I porte un substituant en position 4.

5. Complexes métalliques selon les revendications 1, 2 et 4, caractérisés en ce que le noyau benzénique porte un atome de chlore ou de brome ou un groupe nitro ou méthoxycarbonyle.

6. Utilisation des complexes métalliques selon les revendications 1 à 5 comme pigments.

7. Utilisation des complexes métalliques selon les revendications 1 à 5 pour la pigmentation de vernis.

8. Procédé de préparation des complexes métalliques selon les revendications 1à 5, caractérisé en ce qu'on fait réagir la formyl-3 méthyl-4 dihydroxy-2,6 pyridine, ou l'un de ses sels de métaux alcalins ou de métaux alcalino-terreux, avec un composé ortho-amino-hydroxylique répondant à la formule

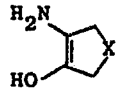

dans laquelle X a la signification donnée à la revendication 1 et le noyau benzénique ou naphtalénique formé par X avec le reset de la molécule peut également être substitué comme indiqué aux revendications 1 à 5, et on métallise le produit de condensation formé.

9. Procédé selon la revendication 8, caractérisé en ce que la réaction et/ou la métallisation sont effectuées dans un solvant organique ou dans l'eau, à des températures comprises entre 60°C et la température de reflux du solvant.

10. Procédé selon les revendications 8 ou 9, caractérisé en ce qu'on effectue la réaction et/ou la métallisation dans une atmosphère de gaz inerte.

11. Procédé selon les revendications 8 à 10, caractérisé en ce qu'on maintient un pH compris entre 5 et 6 lors de la métallisation.

**Claims**

1. Metal complex compounds of the formula I

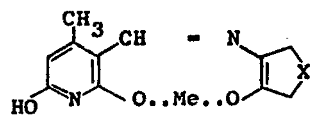 (I)

wherein X stands for

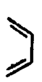 , 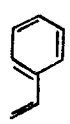 , 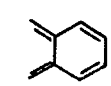 or 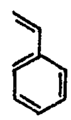

and the benzene or naphthalene ring formed by X and the remaining portion of the molecule may also be substituted with one or two substituents of the methyl, methoxy, chloro, bromo, trifluoromethyl, nitro, cyano, $—CONR_1R_2$ or $—COOR_3$ series, $R_1$ and $R_2$ standing for hydrogen or alkyl with 1 to 4 carbon atoms and $R_3$ for alkyl with 1 to 4 carbon atoms, and Me denotes a nickel, copper, zinc or cobalt atom.

2. Metal complex compounds of the formula II

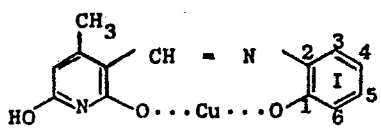 (II)

wherein the benzene ring I may be substituted, as stated in claim 1.

3. Metal complex compounds of claim 2, characterized in that the benzene ring I is unsubstituted.

4. Metal complex compounds of claims 1 and 2, characterized in that the benzene ring I is substituted in the 4-position.

5. Metal complex compounds of claims 1, 2 and 4, characterized in that the benzene ring is substituted by chlorine, bromine, nitro or methoxy carbonyl.

6. The application of the metal complex compounds of claims 1 to 5 as pigments.

7. The application of the metal complex compounds of claims 1 to 5 for pigmenting lacquers and varnishes.

8. A process for the production of the metal complex compounds of claims 1 to 5, characterized in that 3-formyl-4-methyl-2,6-dihydroxy-pyridine or one of its alkali metal salts or alkaline earth salts is reacted with an ortho-amino-hydroxy compound of the formula

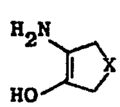

wherein X has the meaning defined in claim 1 and the benzene or naphthalene ring formed by X and the remaining portion of the molecule may also be substituted, as stated in claims 1 to 5, and in that the condensation product obtained is metallized.

9. A process according to claim 8, characterized in that the reaction and/or metallization is carried out in an organic solvent or in water at temperatures between 60°C and the reflux temperature of the solvent.

10. A process according to claim 8 or 9, characterized in that the reaction and/or metallization is carried out in an atmosphere of an inert gas.

11. A process according to claims 8 to 10, characterized in that in the metallization a pH value between 5 and 6 is maintained.